**Europäisches Patentamt**

**European Patent Office**

**Office européen des brevets**

(19)

(11) Numéro de publication : **0 511 086 A1**

## (12) DEMANDE DE BREVET EUROPEEN

(21) Numéro de dépôt : **92401128.1**

(51) Int. Cl.⁵ : **A61B 17/22**

(22) Date de dépôt : **22.04.92**

(30) Priorité : **22.04.91 FR 9104909**

(43) Date de publication de la demande :
**28.10.92 Bulletin 92/44**

(84) Etats contractants désignés :
**AT BE CH DE DK ES GB GR IT LI LU MC NL PT SE**

(71) Demandeur : **ECLIPSE SURGICAL TECHNOLOGIES, Inc.**
**1049 Kiel Court**
**Sunnyvale, California 94089 (US)**

(72) Inventeur : **Boussignac, Georges**
**1 Avenue de Provence**
**F-92160 Antony (FR)**
Inventeur : **Labrune, Jean-Claude**
**19A rue Massenet**
**F-92310 Sèvres (FR)**

(74) Mandataire : **Bonnetat, Christian**
**CABINET BONNETAT 23, Rue de St.Pétersbourg**
**F-75008 Paris (FR)**

(54) **Cathéter destiné à être introduit dans un canal corporel.**

(57)     — La présente invention concerne un cathéter destiné à être introduit dans un canal corporel, comportant un tube (2) dont l'extrémité distale (2a) est associée à au moins une structure radialement déformable (4), elle-même associée à une pluralité de conducteurs linéaires d'énergie (6) reliés à l'extrémité proximale du tube.

— Selon l'invention, au voisinage de leurs extrémités distales, lesdits conducteurs linéaires d'énergie (6) sont logés, de façon au moins sensiblement jointive et en étant libres de se déplacer transversalement les uns par rapport aux autres, dans un espace annulaire (7) formé entre ladite structure radialement déformable (4) et une gaine externe souple (9), de sorte que, quelle que soit la déformation de ladite structure radialement déformable, l'aire de la section transversale dudit espace annulaire reste constante.

FIG.2

FIG.4

La présente invention concerne un cathéter destiné à être introduit dans un canal corporel.

Quoique non exclusivement, les applications du cathéter selon l'invention ont trait plus particulièrement à l'examen des canaux corporels et/ou à la destruction d'obstacles au sein d'un canal corporel, tel qu'un vaisseau sanguin.

On connaît, notamment par le document FR-A-2 651 440, un cathéter comportant un tube dont l'extrémité distale est associée à au moins une structure radialement déformable, elle-même associée à une pluralité de conducteurs linéaires d'énergie reliés à l'extrémité proximale du tube. Ces conducteurs linéaires d'énergie (fibres optiques), reliés à un appareil à laser, permettent de détruire progressivement, par exemple, un athérome formé dans une artère, sous l'action du rayonnement laser véhiculé par les fibres. Cependant, dans ce cas, la structure déformable est préformée pour définir, lorsqu'elle est expansée, une partie cylindrique de diamètre constant susceptible de venir au contact de la paroi interne du canal corporel. En pratique, un tel cathéter est donc adapté à un diamètre défini de canal corporel et, par suite, inutilisable pour d'autres canaux corporels de diamètre différent. De plus, les fibres uniquement réparties, dans ce cas, à l'extrême périphérie de la structure déformable, c'est-à-dire à proximité de la paroi interne du canal corporel, ne peuvent attaquer efficacement que la base de l'athérome, ce qui peut nuire au bon déroulement de l'opération et/ou être inadapté dans le cas où l'intervention, pour telle ou telle raison, doit être réalisée sur une partie plus centrale du canal. Par ailleurs, dans le cathéter du brevet FR-A-2 651 440, les fibres sont noyées dans un manchon relativement rigide enveloppant le tube et la structure radialement déformable, en étant régulièrement réparties angulairement les unes par rapport aux autres, de sorte que, après expansion de la structure radialement déformable, la densité d'énergie fournie par les fibres peut devenir insuffisante.

La présente invention a pour but d'éviter ces inconvénients, et concerne un cathéter dont le diamètre de l'extrémité distale peut être facilement adapté au diamètre du canal corporel dans lequel le cathéter est introduit et/ou modifié conformément à l'intervention souhaitée, tout en conservant, pour le tir laser, une densité d'énergie suffisante.

A cet effet, le cathéter destiné à être introduit dans un canal corporel, du type indiqué ci-dessus, est remarquable, selon l'invention, en ce que, au voisinage de leurs extrémités distales, lesdits conducteurs linéaires d'énergie sont logés, de façon au moins sensiblement jointive et en étant libres de se déplacer transversalement les uns par rapport aux autres, dans un espace annulaire formé entre ladite structure radialement déformable et une gaine externe souple, de sorte que, quelle que soit la déformation de ladite structure radialement déformable, l'aire de la section transversale dudit espace annulaire reste constante. En particulier, lesdits conducteurs linéaires d'énergie sont des fibres optiques, reliées à un appareil à laser.

Ainsi, quelle que soit la déformation de la structure radialement déformable, la densité d'énergie (surface de tir) fournie par les fibres reste constante, ce qui assure l'efficacité de l'intervention, et cela dans une plage de diamètres du canal corporel limitée uniquement par la déformabilité de ladite structure.

Avantageusement, une gaine interne souple est prévue entre lesdits conducteurs et ladite structure radialement déformable, et, de préférence, ladite structure radialement déformable est constituée par un ballonnet gonflable, indépendant de ladite gaine interne.

Selon une autre caractéristique de l'invention, lesdites fibres optiques sont noyées dans un gel ou une mousse de matière synthétique, notamment un silicone.

Par ailleurs, conformément à une variante de l'invention, lesdites fibres optiques peuvent être liées à une membrane s'étendant longitudinalement le long dudit tube, et dont la section présente la forme d'une spirale.

De préférence, dans ledit tube, est prévu un guide métallique s'étendant au-delà de l'extrémité distale du cathéter.

Selon encore une autre caractéristique de l'invention, ladite gaine externe est ouverte à l'extrémité distale du cathéter, de façon à dégager la surface de tir des conducteurs linéaires d'énergie.

Avantageusement, ladite gaine externe est réalisée en un matériau radioopaque.

Les figures du dessin annexé feront bien comprendre comment l'invention peut être réalisée.

La figure 1 est une coupe longitudinale partielle d'un exemple de réalisation du cathéter selon l'invention dans l'état rétracté du ballonnet gonflable.

La figure 2 est une coupe transversale, selon la ligne II-II, du cathéter de la figure 1.

La figure 3 est une vue analogue à la figure 1, montrant le cathéter dans un état expansé du ballonnet gonflable.

La figure 4 est une coupe transversale, selon la ligne IV-IV, du cathéter de la figure 3.

Les figures 5 et 6 illustrent, en coupe transversale, un autre exemple de réalisation du cathéter de l'invention, respectivement dans l'état rétracté et dans un état expansé du ballonnet gonflable.

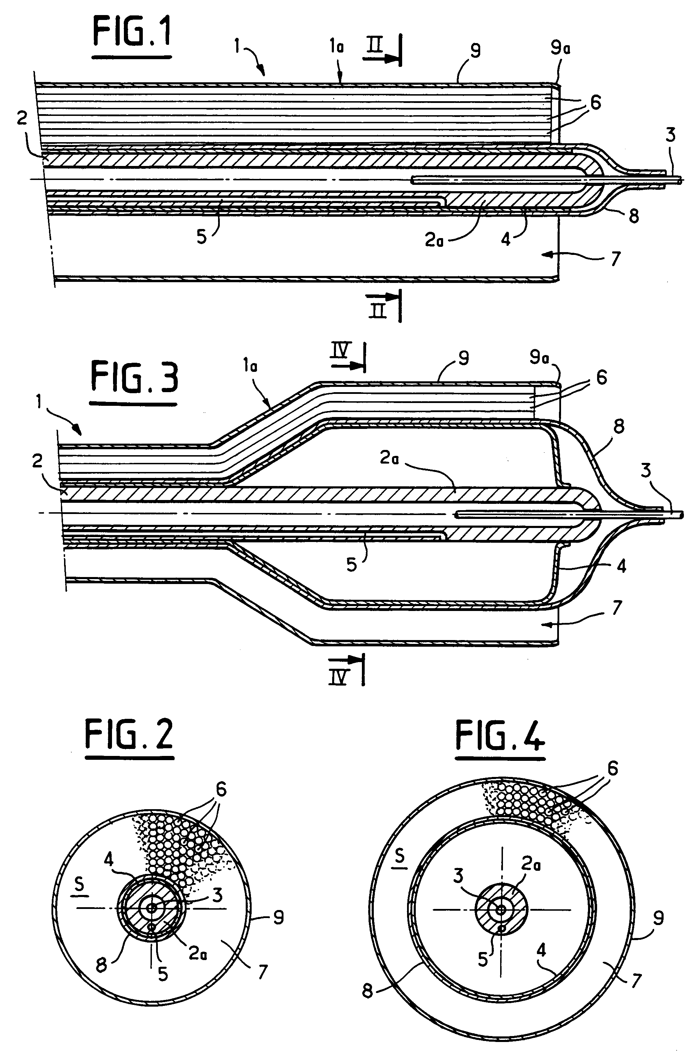

Le cathéter 1, dont seule l'extrémité distale 1a est représentée sur les figures 1 et 3, est destiné à être introduit dans un canal corporel (non représenté) tel que, par exemple, un vaisseau sanguin.

Le cathéter 1 comporte un tube central 2 à l'intérieur duquel est agencé, dans cette réalisation, un guide métallique 3, permettant d'assurer le centrage du cathéter dans le canal corporel, et se prolongeant pour cela, lorsque le cathéter est introduit dans ledit canal, au-delà de l'extrémité distale de celui-ci. Au voisinage de l'extrémité distale 2a du tube 2, est prévue une structure radialement déformable, entourant le tube, constituée d'un ballonnet gonflable 4, dont le gonflage est obtenu par amenée de fluide par le conduit 5 noyé dans la paroi du tube 2.

Par ailleurs, une pluralité de fibres optiques 6 sont logées, au moins au voisinage de leurs extrémités distales, dans un espace annulaire 7 formé entre une gaine interne souple 8, adjacente au ballonnet 4, et une gaine externe souple 9, de sorte que, quelle que soit l'état de gonflage du ballonnet 4, l'aire S de la section transversale de l'espace annulaire 7 reste constante. Le ballonnet 4 est indépendant de la gaine interne 8, c'est-à-dire qu'il n'est pas lié à celle-ci, et la pression du ballonnet est transmise aux fibres 6 par l'intermédiaire de la gaine interne 8.

Les fibres 6 peuvent être des fibres optiques pures ou des fibres optiques conductrices d'énergie en étant pour cela reliées à un appareil à laser (non représenté), ou une combinaison des deux. Le praticien peut ainsi, d'une part, examiner la zone souhaitée du canal corporel, tel qu'un vaisseau sanguin, et notamment sa paroi interne, et il peut, d'autre part, procéder à la destruction d'une formation athéromateuse avec efficacité et précision, dans la mesure où, comme déjà indiqué, la densité d'énergie (surface de tir) fournie par les fibres reste constante, et cela dans une plage de diamètres du cathéter uniquement limitée par la déformabilité du ballonnet (et bien sûr le diamètre du canal corporel). On notera, à ce propos, que l'état expansé du ballonnet gonflable, montré sur la figure 3, ne représente qu'un exemple d'expansion possible parmi d'autres. Par ailleurs, un tel agencement garantit la conservation du parallélisme des fibres quel que soit l'état d'expansion du ballonnet.

Comme montré sur les figures 1 et 3, les gaines interne 8 et externe 9 sont ouvertes à leur extrémité distale, notamment la gaine externe 9 pour dégager ainsi la zone de tir correspondant à l'espace annulaire 7. De plus, l'extrémité libre 9a de la gaine externe 9 est arrondie et légèrement courbée vers l'intérieur pour éviter d'endommager la paroi du canal corporel. Par ailleurs, la gaine externe 9, notamment, peut être réalisée en un matériau radioopaque pour localiser le cathéter dans le canal corporel.

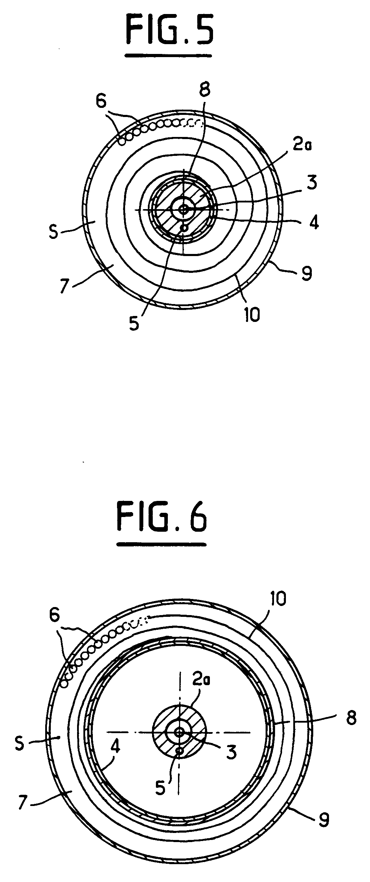

Comme cela résulte en particulier de l'examen des figures 2 et 4, les fibres 6 sont agencées dans l'espace annulaire 7 de façon au moins sensiblement jointive et en étant libres de se déplacer transversalement les unes par rapport aux autres, c'est-à-dire que, lorsque le ballonnet 4 passe de l'état rétracté de la figure 2 à l'état expansé de la figure 4, les fibres 6 se réarrangent comme illustré, le nombre de "couches" radiales de fibres diminuant mais l'aire S de la surface de tir restant constante. Il est bien entendu que le nombre de couches de fibres illustré dans l'un et l'autre cas n'est donné qu'à titre d'exemple, et qu'il peut varier dans une large mesure pourvu que, dans l'état le plus expansé du ballonnet, il reste au moins une couche de fibres. En d'autres termes, lors du gonflage du ballonnet, les fibres d'une couche initiale s'écartent et les espaces inter-fibres ainsi créés sont occupés par des fibres de la couche initialement sous-jacente.

Dans l'exemple de réalisation illustré par les figures 1 à 4, les fibres optiques 6 peuvent être noyées dans un gel ou une mousse de matière synthétique, notamment un silicone, qui n'entrave pas le libre déplacement transversal des fibres lors du gonflage du ballonnet.

Par ailleurs, comme on le voit sur les figures 5 et 6, conformément à une variante de l'invention où l'on retrouve le tube 2, le ballonnet 4 et les gaines interne 8 et externe 9, les fibres 6 sont liées à une membrane 10 s'étendant longitudinalement le long du tube 2, et dont la section présente une forme de spirale qui, comme illustré, se déforme lors du gonflage du ballonnet 4, l'aire S de la section transversale de l'espace annulaire 7 restant de même constante quel que soit l'état d'expansion dudit ballonnet.

On notera de plus que des conduits de liquide d'amorçage de conduction laser et/ou de nettoyage peuvent être prévus parmi les fibres 6. On peut également associer au tube 2 des moyens de visualisation angioscopique.

## Revendications

1 - Cathéter destiné à être introduit dans un canal corporel, du type comportant un tube dont l'extrémité distale est associée à au moins une structure radialement déformable, elle-même associée à une pluralité de conducteurs linéaires d'énergie reliés à l'extrémité proximale du tube,
caractérisé en ce que, au voisinage de leurs extrémités distales, lesdits conducteurs linéaires d'énergie (6) sont logés, de façon au moins sensiblement jointive et en étant libres de se déplacer transversalement les uns par rapport aux autres, dans un espace annulaire (7) formé entre ladite structure radialement déformable (4) et une gaine externe souple (9), de sorte que, quelle que soit la déformation de ladite structure radialement déformable, l'aire de la section transversale dudit espace annulaire reste constante.

2 - Cathéter selon la revendication 1, caractérisé en ce que lesdits conducteurs linéaires d'énergie sont des fibres optiques (6), reliées à un appareil à laser.

3 - Cathéter selon la revendication 1 ou la revendication 2, caractérisé en ce qu'une gaine interne souple (8) est prévue entre lesdits conducteurs (6) et ladite structure radialement déformable (4).

4 - Cathéter selon la revendication 3,

caractérisé en ce que ladite structure radialement déformable est constituée par un ballonnet gonflable (4), indépendant de ladite gaine interne (8).

5 - Cathéter selon l'une quelconque des revendications 2 à 4, caractérisé en ce que lesdites fibres optiques (6) sont noyées dans un gel ou une mousse de matière synthétique.

6 - Cathéter selon la revendication 5, caractérisé en ce que ladite matière synthétique est un silicone.

7 - Cathéter selon l'une quelconque des revendications 2 à 6, caractérisé en ce que lesdites fibres optiques (6) sont liées à une membrane (10) s'étendant longitudinalement le long dudit tube (2), et dont la section présente la forme d'une spirale.

8 - Cathéter selon l'une quelconque des revendications 1 à 7, caractérisé en ce que, dans ledit tube (2), est prévu un guide métallique (3) s'étendant au-delà de l'extrémité distale du cathéter.

9 - Cathéter selon l'une quelconque des revendications 1 à 8, caractérisé en ce que ladite gaine externe (9) est ouverte à l'extrémité distale du cathéter, de façon à dégager la surface de tir des conducteurs linéaires d'énergie (6).

10 - Cathéter selon l'une quelconque des revendications 1 à 9, caractérisé en ce que ladite gaine externe (9) est réalisée en un matériau radioopaque.

FIG.1

FIG.3

FIG.2

FIG.4

# FIG. 5

# FIG. 6

央 EP 0 511 086 A1

| | Office européen des brevets | **RAPPORT DE RECHERCHE EUROPEENNE** | Numero de la demande |
|---|---|---|---|

EP    92 40 1128

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 387 754 (CARL-ZEISS-STIFTUNG)<br>--- | | A61B17/22 |
| A | DE-A-2 106 470 (MÜSSIGANG)<br><br>----- | | |

**DOMAINES TECHNIQUES RECHERCHES (Int. Cl.5)**

A61B

Le présent rapport a été établi pour toutes les revendications

| Lieu de la recherche | Date d'achèvement de la recherche | Examinateur |
|---|---|---|
| LA HAYE | 17 JUIN 1992 | BARTON S. |